Europäisches Patentamt

(19) European Patent Office

Office européen des brevets

(11) Publication number: **0 215 923**
B1

(12) **EUROPEAN PATENT SPECIFICATION**

(45) Date of publication of patent specification: **27.12.90**

(21) Application number: **86902215.2**

(22) Date of filing: **20.03.86**

(86) International application number:
**PCT/US86/00577**

(87) International publication number:
**WO 86/05514 25.09.86 Gazette 86/21**

(51) Int. Cl.⁵: **C 12 N 15/00, C 12 N 1/00, C 12 N 5/00**

(54) **EXPRESSION OF tPA IN MAMMALIAN CELLS.**

(30) Priority: **22.03.85 US 715237**

(43) Date of publication of application:
**01.04.87 Bulletin 87/14**

(45) Publication of the grant of the patent:
**27.12.90 Bulletin 90/52**

(84) Designated Contracting States:
**AT BE CH DE FR GB IT LI LU NL SE**

(56) References cited:
EP-A-0 173 552
EP-A-0 211 260

Proceedings National Academy Sciences U.S.A.,
vol. 81, issued September, 1984, Ny et al, "The
Structure of the Human Tissue-Type
Plasminogen Activator Gene: Correlation of
Intron and Exon Structures to Function and
Structural Domains", pp. 5355-5358

(73) Proprietor: **CHIRON CORPORATION**
**4560 Horton Street**
**Emeryville California 94608 (US)**

(72) Inventor: **HAIGWOOD, Nancy**
**7050 Sayre Drive**
**Oakland, CA 94611 (US)**

(74) Representative: **Goldin, Douglas Michael et al**
**J.A. KEMP & CO. 14, South Square Gray's Inn**
**London WC1R 5EU (GB)**

(56) References cited:
Molecular and Cellular Biology, volume 2, no.
11, issued in November 1982, Kaufman et
al.:"Construction of a Modular Dihydrofolate
Reductase cDNA Gene: Analysis of Signals
Utilized for Efficient Expression", pp.
1304,1305,1314,1317

Molecular and Cellular Biology, volume 5, no. 7,
issued July, 1985, Kaufman et al.:"Coexpression
of Human Tissue-Type Plasminogen Activator
and Murine Dihydrofolate Reductase Sequences
in Chinese Hamster Ovary Cells", pp. 1750-1752,
1757,1758

## EP 0 215 923 B1

(56) References cited:
Journal of Biological Chemistry, vol. 260, no. 20, issued September 15, 1985, Fisher et al.:"Isolation and Characterization of The Human Tissue-Type Plasminogen Activator Structural Gene Including Its 5' Flanking Region", pp. 11223-11230

Nature, volume 301, issued January 1983, Pennica et al.:"Cloning and Expression of Human Tissue-Type Plasminogen Activator cDNA in E.coli", pp. 214,215,217,219,220

Science, volume 220, issued June 10, 1983, Craik et al.:"Splice Junctions: Association with variation in Protein Structure", pp. 1125-1129

**Description**

## BACKGROUND OF THE INVENTION

Field of the Invention

There is an increasing number of genes being introduced into foreign hosts for expression. A substantial proportion of the genes which are employed are obtained from cDNA. In those instances where the natural gene has one or more introns, it has frequently been found to be much more convenient to employ the cDNA rather than handling larger pieces of DNA which involve the intron and add further complexity to the manipulation of the gene in conjunction with the regulatory signals associated with expression and replication.

The function of introns is still not well established. While much progress has been made in an understanding of the splicing mechanism involved in removing the introns, the role of introns in the process of transcription and translation has not been explained. The fact that cDNA can be readily expressed establishes that introns are not essential for the expression of a coding sequence.

An important factor in the preparation and production of polypeptides and proteins in cellular hosts is the efficiency with which the polypeptide or protein is produced. Since nutrients must be supplied to the host to maintain its viability, the nutrients are used primarily for the growth and replication of the host, rather than the product of interest. It thus becomes important to maximize utilization of the nutrients for the production of the desired product, as well as enhancing the proportionate amount of the desired product as compared to the total protein produced, which greatly aids in the efficiency with which the desired product may be isolated in pure form.

Description of the Prior Art

Pennica et al., Nature (1983) 301:214—221; UK Patent Appln. No. GB 2,119,804 describe the isolation and cloning of a cDNA for human tissue plasminogen activator. Ny et al., Proc. Nat'l Acad. Sci. USA (1984) 81:5355—5359 describe the human tissue-type plasminogen activator gene and the exon-intron relationship. Hamer and Leder, Cell (1979) 18:1299—1302, suggest that introns aid in the stability of RNA, while Gething and Sambrook, "The Expression of the Influenza Virus Hemagglutinin Gene from SV—40—HA Recombinants" in Eukaryotic Viral Vectors, ed., Y. Gluzman, pp. 29—33 (1982), indicate the opposite experience. Kaufman and Sharp, Molec. and Cell Biol. (1982) 2: 1304—1319 show that a hybrid intron can be used in the expression of dihydrofolate reductase cDNA. Lau and Kan, Proc. Nat'l Acad. Sci. USA (1983) 80:5225—5229, describe cosmid vectors and their use in COS cells and permanent cell lines.

## SUMMARY OF THE INVENTION

Improved expression of tissue plasminogen activator is obtained in mammalian cells by employing a coding sequence for tissue plasminogen activator (tPA) which is interrupted by at least one intron. Either the wild-type tissue plasminogen activator gene or a chimeric gene is employed which codes for the desired protein product. Active promoters are employed which result in enhanced yields of tissue plasminogen activator.

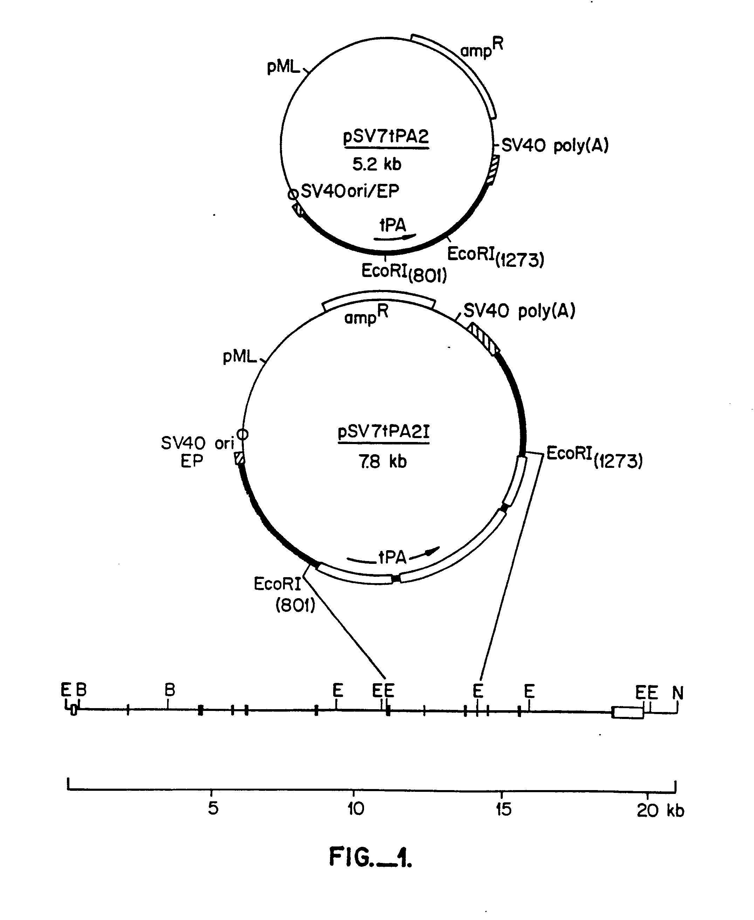

## BRIEF DESCRIPTION OF THE DRAWING

The Figure depicts two related plasmids, one being an embodiment of the subject invention.

At the top of the Figure is shown a schematic diagram of the tPA cDNA expression plasmid pSV7tPA2. Hatched regions indicate 5' and 3' untranslated sequences and black regions indicate the coding sequences for preprotPA. The SV40 origin is noted as a circle, and the approximate locations of the SV40 early promoter (EP) and early region polyadenylation sequences (poly(A)) are noted. The EcoRI sites at positions 801 and 1273 are also indicated. Below this plasmid is the plasmid bearing the chimeric tPA gene, pSV7tPA2I. As above, black regions indicate coding sequences, interrupted by introns (white boxed regions) from the genomic tPA gene.

A schematic diagram of the genomic tPA gene is drawn at the bottom to indicate the region excised from the genomic clone to replace the cDNA between positions 801 and 1273. Restriction sites for EcoRI (E), BamHI (B) and NruI (N) are marked on the genomic map.

## DESCRIPTION OF THE SPECIFIC EMBODIMENTS

DNA constructions and their use for improved expression of tissue plasminogen activator (tPA) are provided where the DNA constructions include a coding sequence for mammalian tissue plasminogen activator, where the coding sequence is interrupted by at least one intron. The genomic gene or chimeric gene lacking one or more of the naturally-occurring introns is joined to transcriptional regulatory sequences functional in the mammalian host.

The gene which is employed for encoding the tissue plasminogen activator may encode for mature tissue plasminogen activator, a precursor or proform of tissue plasminogen activator, which intends the loss of an N-terminal sequence, or a pre-proform involving a leader sequence which provides for secretion.

The tPA coding sequence which is employed will be characterized by having at least one intron of at least about 100bp, more usually at least about 300bp, and not more than about 5kbp, usually not more than about 4kbp. The introns which are employed may be the naturally-occurring introns associated with the tissue plasminogen activator gene or introns derived from a different mammalian gene, such as beta- globin, or hybrid introns such as the adenovirus-immunoglobulin hybrid intron used to express a *dhfr* cDNA as described by Kaufman and Sharp, *supra*.

Desirably, native introns are employed and at their native site. The gene will usually have fewer than the natural number of introns, the gene may have from 1 to 13 introns, usually 2 to 13 introns, preferably from 2 to 6 introns or from 9 to 13 introns, particularly among the introns "F" to "M", using the designations of Ny *et al.*, *supra*, which disclosure is incorporated herein by reference. The DNA fragment encoding the human tissue plasminogen activator gene and including the introns will be at least about 2.5kbp and not more than about 30kbp. The introns will have the appropriate splicing signals at their termini.

A large number of different promoter regions may be employed which are active in mammalian cells. Heterologous promoters may be obtained from mammalian viruses, such as SV40 (early or late) adenovirus (early or late) or retroviruses (LTRs). Alternatively, regulatable promoters such as those from mouse or human metallothioneins I and II, heat shock proteins, etc. may be employed. Descriptions of these promoters may be found in Hamer *et al.* (1983) in Eukaryotic Viral Vectors, Y. Gluzman *ed.* pp 7—12, Mayo *et al.*, *Cell* (1982) *29*:99—108 and Karin *et al.*, *Nature* (1984) *308*:513—519.

Any convenient polyadenylation site may be used, such as one associated with the promoter region, native to the tissue plasminogen activator gene, or from another mammalian gene.

Of particular interest is the combination of the SV—40 enhancer and early promoter region, as well as replication system, but lacking the T-antigen, in combination with COS cells, which contain the T-antigen.

The tissue plasminogen activator gene may be obtained from any mammalian host, particularly primate (e.g., human), bovine, equine, canine, porcine, feline, rodentia, or the like.

Once an expression cassette has been prepared containing the 5' and 3' untranslated regions containing transcriptional and translational initiation and termination regulatory sequences usually flanking the intron-containing gene, the cassette may then be joined to a vector for transformation into a mammalian cell host. In some instances transcriptional regulatory regions, e.g., enhancers, may be included in an intron.

The particular order in which the parts are brought together is not a critical element of the subject invention, so that the flanking regions might be first bound to a replication system including a marker or other regions, such as enhancers, transcriptional regulatory regions, or the like, prior to insertion of the gene. The manner in which the various fragments are brought together will depend on a number of factors related to ease of construction, choice of restriction sites, availability of particular fragments, and the like.

In part, the choice of replication system will depend upon whether one wishes to obtain transient or stable expression. For transient expression, episomal elements based on viral sequences (e.g., SV—40), containing an origin of replication are used. Stable expression can be achieved using episomal elements (e.g., bovine papilloma virus based vectors) or sequences integrated into the genome.

A number of these viral sequences have been joined to markers, such as the genes *gpt, neo* and *dhfr*. These markers allow for selection of cells containing the vector and for amplification of the integrated foreign sequences. If desired, to further enhance the production of the tissue plasminogen activator, one may amplify the gene by preparing a tandem construct where the tissue plasminogen activator cassette is in tandem with an expression cassette of a gene such as *dhfr*, metallothionein, or the like. Alternatively, cells may be cotransformed with one construct containing the tPA expression cassette and a second independent construct containing the selectable marker.

The host may be any convenient mammalian cell which is capable of splicing and expressing the tissue plasminogen activator efficiently in culture. A wide variety of cells exist such as CHO cells, COS cells, LTK cells, HeLa, and CV—1. These cells are merely set forth as illustrative of well-established cell lines, and are not intended to limit the subject invention to any particular established cell line, but other cell lines presently established or established in the future may be advantageously employed.

The genomic tissue plasminogen activator gene may be obtained as described by Ny *et al.*, *supra*, and the cDNA gene may be obtained as described by Pennica, *supra*, or in UK Patent Application No. 2,119,804. These sequences may then be used in a variety of ways. The entire gene may be used and inserted into an expression vector having the appropriate regulatory sequences to provide the expression cassette joined to a replication system or DNA which will aid integration into the host genome or both. Alternatively, based on the restriction maps provided by Ny *et al.*, *supra*, and Pennica *et al.*, *supra*, various fragments may be obtained from the cDNA and genomic DNA gene sequences and joined together to provide hybrid genes, where a portion is from the cDNA gene and a portion from the genomic gene. The hybrid gene may be prepared by the substitution of a portion of the genomic gene with a cDNA fragment, or *vice versa*, where the hybrid gene terminates with either the cDNA or the genomic DNA or the 5'-proximal portion of one of the tissue plasminogen activator genes is joined to the 3'-proximal portion of the other gene.

The expression cassette may be introduced into the host by any convenient means, such as calcium-phosphate-precipitated DNA, transfection, transduction, or the like. The particular manner is not critical to this invention, and once the DNA has been introduced into the host cell, the procedure need not be repeated. Therefore, while high efficiencies of introduction of the expression cassette into the host is desirable, it is not necessary.

The mammalian cells may then be grown in an appropriate medium, such as Dulbecco's Modified Eagle Medium (DMEM) containing 10% to 20% fetal calf serum or other medium in which these cells may be stably maintained. Depending upon whether the leader sequence has been retained and the product is capable of secretion, where the product is produced extracellularly, the medium may be continuously or repetitively exchanged and the tissue plasminogen activator protein or proform isolated from the medium. Where the leader is not retained and the tissue plasminogen activator is maintained intracellularly, the cells may be harvested, killed and lysed, and the tissue plasminogen activator isolated.

Various techniques for isolation and purification are known, such as affinity chromatography, HPLC, reverse phase HPLC, electrophoresis, extraction, gel permeation chromatography, and the like. By employing the expression constructs of the subject invention, the yields of tissue plasminogen activator can be substantially enhanced as compared to employing the cDNA gene, which has been previously employed both in prokaryotic and eukaryotic cells.

The following examples are offered by way of illustration and not by way of limitation.

## EXPERIMENTAL

### 1. Construction of Expression Vectors Containing a tPA Hybrid Gene

A human genomic DNA library consisting of a HaeIII partial digest of human DNA cloned into bacteriophage lambda Charon 4A at the EcoRI sites (available from T. Maniatis) was screened with a tPA cDNA clone 23 which comprises approximately bases 1270 to 2540 of the cDNA sequence as numbered by Pennica et al. supra. Approximately one million plaques were screened, yielding 12 clones which were positive for tPA sequences. Further screening with three tPA oligomers corresponding to nucleotides 76 to 95; 1129 to 1148 and 1174 to 1793, respectively, which correspond to the 5', central and 3' regions of the Pennica cDNA sequence was done. This screening identified two clones, λgtPA8 and λgtPA9 which hybridized with all three probes. The sequence was mapped and found to be in agreement with the published tPA genomic clone, Ny et al., supra. The 5' end of the subject genomic clone contains the complete second exon encoding 26bp of 5'-untranslated sequence plus at least 30bp of the preceding intron. All of the remaining tPA sequences up to and including the polyadenylation site are present.

An M13 subclone containing a genomic EcoRI fragment spanning positions 801 to 1273 in the cDNA, but containing 2.6kb of genomic sequence was characterized in detail by restriction endonuclease mapping and partial DNA sequence analysis. By these analyses, the two EcoRI sites at the ends of the clone were shown to be the two sites which exist in the cDNA. By substituting the genomic EcoRI fragment for the EcoRI fragment of the cDNA, a hybrid gene containing three natural introns would be obtained.

The following procedure was used for the preparation of expression vectors containing the hybrid gene: plasmid pSV7tPA2 was constructed by excising the full length tPA cDNA from an M13 cloning vector by digestion with HindIII, the overhang filled in by the Klenow fragment of DNA polymerase I and dNTPs, followed by digestion with BalI. The resulting about 2100bp fragment was gel purified. pSV7b, a pML (Lusky and Botchan, Nature (1981) 293:79—81) based plasmid contains the SV40 origin, early promoter and polyadenylation site and a polylinker including BglII and SmaI sites between the promoter and polyadenylation site. The plasmid was digested with BglII and SmaI where the BglII site was filled in with DNA polymerase I (Klenow fragment) and dNTPs. The 2100 bp tPA cDNA was ligated to pSV7b, the ligated DNA was transformed into competent E. coli HB101, ampicillin-resistant clones selected and grown, and small-scale DNA preparations screened for the correct insert. Fine structure restriction mapping confirmed that the tPA cDNA and flanking sequences were not detectably deleted or rearranged. The DNA sequence was also established for the 5' end of the tPA insert. The resulting plasmid is referred to as pSV7tPA2.

pSV7tPA2 was digested with EcoRI, and the 2.6 kb genomic EcoRI fragment spanning cDNA positions 801 to 1273 described above was substituted into the cDNA. The resulting plasmid pSV7tPA2I was isolated and characterized by restriction mapping to establish that the correct structure was present.

### 2. Assays for tPA

There are two types of assays used for tPA, both described by Granelli-Piperno and Reich, (1978) J. Exp. Med. 148:223—234. The first of these is used to assay live cells directly using a casein-agar-plasminogen overlay. When used on COS cells or CHO cells, the assay allows identification of cells which have been transfected with the tPA expression plasmid and which are expressing the gene at a level detected by the assay. Since the transfection efficiencies of the two types of cells are documented and constant (5—15% for COS cells and $1 \times 10^{-4}$ per µg for CHO cells), the assay measures the relative expression efficiency of different constructions. The results are seen as a difference in number and size of the cleared zones over the cells expressing tPA.

Agar overlays containing milk, plasminogen, and nutrient medium without serum are prepared as follows (per 6 cm dish):

0.2 ml 2.5% Difco agar
0.125 ml 8% milk (boiled 30 minutes in $H_2O$ bath)
0.25 ml DMEM or 0.25 ml of 2-fold concentrated (2x) DMEM
0.025 ml 7.5 U/ml plasminogen, sterile filtered

Components are mixed in order at 45°—50°C and pipetted onto cells (from which media has been aspirated). Cells are monitored for the production of tPA by inspecting for clear spots in the cloudy overlay.

Photographs are taken after 2—6 hours of incubation at 37°C. COS cell assays are recorded by photography and counting of cleared zones; CHO cells are scored for number of cleared zones and individual clones are picked for expansion directly from the assay plate to microwell plates.

The second version of the assay is quantitative and is used to measure the tPA production per cell of permanent expressor lines of CHO cells. Supernatants are removed from cultures of producing cells after 24 or 48 hours of incubation, and cells are removed by trypsinization and counting in a hemacytometer. The supernatants are used undiluted or diluted and are applied to holes punched in casein-agarose-plasminogen plates. The size of the radial zone of clearing is proportional to the concentration of tPA in the sample.

Casein-plasminogen-agarose plates are prepared by mixing 4.25 ml agarose (3.5%), 6.2 ml 2x DMEM with no added serum, 3.75 ml milk (8% nonfat dry milk powder in water, heated 30 min. in boiling water bath), and 0.2 ml plasminogen (7.5 U per ml) at 48°C and pipetting the mixture onto glass plates to achieve a gel of uniform thickness and smooth surface. After cooling for approximately 5 minutes to harden, holes are punched into the agar at regular intervals using a metal punch, and the holes are removed by aspiration. Plates thus prepared are stored in a humid atmosphere at 4° until use (within 8 hours). Samples are applied using a Pipetteman, 5 microliters per well. The urokinase stock (1700 IU per vial) is dissolved in 340 microliters of distilled water or PBS to a final concentration of 5,000 IU per ml. This stock is diluted in PBS or DMEM (no difference in these buffers) by adding 4 µl of the stock (5,000 IU per ml) to 16 µl of diluent (1/5 dilution). Ten µl of the 1/5 stock is added to 90 µl of diluent for a 1/50 dilution. Fifty µl of the 1/50 dilution is added to 50 µl of diluent for the 1/100 dilution, and all subsequent 2-fold dilutions are done in this manner. Dilutions of urokinase are prepared fresh each day from the stock, which is stored at 4°C in PBS. Standard solutions are monitored for loss of activity by comparison at two week intervals to freshly dissolved urokinase powder. Test samples are diluted 2-fold in the same diluent as the standard. Plates are incubated at 37°C in a humid chamber and monitored as a function of time by Polaroid photography. The sizes measured are converted to actual sizes by use of a metric ruler and relating the photograph size to actual size.

3. Transfections and tPA Expression Levels

COS—7 cells (Gluzman, *Cell* (1981) *23*:175) were transfected with both plasmids pSV7tPA2 and pSV7tPA2I, using a modification of the procedure described by Graham and van der Eb, *Virology* (1973) *52*:456—467. The samples were added to the dishes in duplicate and allowed to settle onto the cells for 6hr in a $CO_2$ incubator (37°C). Six hours later the supernatants were aspirated and the cells rinsed gently with calcium- and magnesium-free phosphate-buffered saline (PBS—CMF). The dishes were exposed to glycerol as an adjuvant for 3.5—4min, rinsed and fed with DMEM medium, supplemented with 4.5 mg/ml glucose, 3.7 mg/ml sodium bicarbonate, 292 µg/ml glutamine, 110 µg/ml sodium pyruvate, 100 units/ml penicillin, 100 units/ml streptomycin, and 10% fetal calf serum (FCS). At various times after the adjuvant shock, the medium was replaced with medium lacking fetal calf serum. Twelve hours after serum withdrawal, cells were assayed for expression of tPA using the casein-plasminogen-agar overlay as previously described. Maximum expression was observed between 36 and 48hr after the start of the transfection.

The number of cleared zones in the dishes transfected by pSV7tPA2I was two to three-fold higher than that with pSV7tPA2 (Table I). Because the transfection frequency is a constant in COS cells, this result signifies better expression in the construction containing the introns. Since the introns were the only differences between the two constructions, the improvement must be attributed to the presence of the introns.

CHO dhfr⁻ cells (Urlaub & Chasin *Proc. Natl. Acad. Sci. USA* (1980) *77*:4216) were plated at a density of $5 \times 10^5$ to $10^6$ cells per 10cm dish the day prior to transfection in nutrient medium (F12 supplemented with 1.18 mg/ml sodium bicarbonate, 292 µg/ml glutamine, 110 µg/ml sodium pyruvate, 100 u/ml penicillin, 100 u/ml streptomycin, 150 µg/ml proline and 10% FCS). The cells were transfected by the same method used to transfect COS cells, except that the tPA expression plasmid (pSV7tPA2 or pSV7tPA2I) was mixed with a plasmid bearing as a selectable marker, a *dhfr* gene driven by the adenovirus major late promoter, and these plasmids were coprecipitated in calcium phosphate. The plasmid bearing the *dhfr* gene was constructed by fusing the major late promoter from adenovirus-2 (Ad—MLP, map units 16—17.3) to the mouse *dhfr* cDNA at the 5' end. DNA coding for the intron for SV40 small t Antigen and the SV40 early region polyadenylation site was obtained from pSV2-neo, described in Southern and Berg, *J. Mol. Appl. Genet.* (1982) *1*:327—341, and fused to the 3' end of the *dhfr* cDNA. These three segments were subcloned into pBR322 to obtain the plasmid Ad-*dhfr*. This plasmid is functionally similar to the *dhfr* plasmid described in Kaufman and Sharp, *supra*. Forty-eight hours after the addition of DNA to the cells, the cells were split 1:20 into selective medium (DMEM supplemented with proline and fetal calf serum as above, or with dialyzed fetal calf serum). After growth in selective medium for 1 to 2 weeks, colonies appeared and were assayed for production of tPA by the casein-plasminogen-agar overlay assay and in casein-agarose-plasminogen plates for quantitation.

Positive clones were more numerous and cleared zones were larger in the plates with cells transfected with pSV7tPA2I than those transfected with pSV7tPA2. Individual clonal lines have been grown up and assayed for production of tPA to compare the expression levels per cell (pg/cell). The results for the lines isolated to date are summarized in Table II.

As shown in Table II, the levels of tPA produced (measured on a per cell basis) are significantly higher in the pSV7tPA2I lines than in the pSV7tPA2 lines. The increase ranges from 1.5 to 16.5-fold.

### TABLE 1
Comparison of transient tPA expression using a cDNA containing plasmid (pSV7tPA2) or an intron (pSV7tpA2I)

| DNA | Clear Zones[1]<br>(for molar equivalents of plasmid) | Average |
|---|---|---|
| None | 0<br>0 | 0<br>0 |
| pSV7tPA2 (+) | 460<br>640 | 550 |
| pSV7tPA2I (+) | 1482<br>1302 | 1392 |

[1] Transfections were done with 7.5 µg of plasmid per dish. Zones were scored 2 hours after addition of the plasminogen-casein-agar overlay to the plates. Overlays were done at 36 hours after the adjuvant shock, 42 hours after addition of the DNA to the cells.

### TABLE II
Comparison of tPA expression between pSV7tPA2 and pSV7tPA2I clones in CHO cells.[1]

| Line | tPA Level |
|---|---|
| pSV7tPA2 Derivatives | (pg/cell) |
| 9—1 | 0.13 |
| 9—4[1] | <0.27 |
| pSV7tPA2I Derivatives | |
| 7—9 | 3.30 |
| 7—15[1] | 0.36 |
| 7—19[1] | 0.77 |
| 7—21 | 4.35 |
| 7—26 | 2.00 |

[1] Based upon nonconfluent cultures.

It is evident from the above results that remarkable enhancements in production of tPA can be achieved by employing a gene containing at least one and preferably a plurality of introns. Since variable results have been obtained previously in the literature concerning the necessity for introns and these results have been interpreted as the introns contributing to messenger RNA stability, the present result of increased expression and higher efficiencies of productive transfection could not have been predicted from the prior art. The subject invention therefore provides an improved method for making an important mammalian protein, tissue plasminogen activator where higher yields can be obtained so as to allow for higher efficiencies in production and purification and greater economies.

Although the foregoing invention has been described in some detail by way of illustration and example for purposes of clarity of understanding, it will be obvious that certain changes and modifications may be practiced within the scope of the appended claims.

Plasmid pSV7tPA2I was deposited on February 14, 1985 and given A.T.C.C. Accession Number 40163.

**Claims**

1. In a method for producing tissue plasminogen activator in a mammalian host, where the gene encoding for the tissue plasminogen activator is under the transcriptional control of other than the natural promoter region, the improvement which comprises:

employing for expression a gene having a coding sequence coding for tissue plasminogen activator where the coding sequence is interrupted by at least one intron of at least about 100bp and is capable of being spliced in said host to provide an mRNA having the proper reading frame.

2. A method according to Claim 1, wherein said gene is a chimeric gene comprised of cDNA and genomic DNA having at least one intron and providing a complete coding sequence with proper reading frame upon splicing.

3. A method according to Claim 2, wherein said tissue plasminogen activator is human and an *Eco*RI—*Eco*RI genomic fragment is substituted into the *Eco*RI—*Eco*RI region of the cDNA coding for human tissue plasminogen activator.

4. A method according to Claim 1, wherein the introns are native introns at the native sites.

5. A method according to Claim 4, having a viral promoter region for regulating transcription of said gene.

6. A DNA construct comprising transcriptional initiation and termination regulatory sequences functional in a mammalian host and at least the initiation sequence being other than the wild-type tissue plasminogen activator initiation sequence and a gene having a coding sequence coding for tissue plasminogen activator, where the gene is interrupted by at least one intron capable of being spliced in said mammalian host to an mRNA having the proper reading frame.

7. A DNA construct according to Claim 6, wherein each of said introns is a naturally occurring intron at its natural site.

8. A DNA construct according to Claim 7, wherein said tissue plasminogen activator is human and an *Eco*RI-*Eco*RI genomic fragment is substituted into the *Eco*RI-*Eco*RI region of a cDNA coding for human tissue plasminogen activator.

9. An expression vector capable of stable maintenance in a mammalian host comprising a DNA construct according to Claim 6.

10. An expression vector capable of stable maintenance in a mammalian host comprising a DNA construct according to Claim 7.

11. An expression vector capable of stable maintenance in a mammalian host comprising a DNA construct according to Claim 8.

12. A mammalian cell in culture containing an expression vector according to Claim 9.

13. A mammalian cell in culture containing an expression vector according to Claim 11.

14. A mammalian cell in culture wherein a DNA construct according to Claim 6 is integrated into a chromosome.

15. A mammalian cell in culture wherein a DNA construct according to Claim 8 is integrated into a chromosome.

**Patentansprüche**

1. Verfahren zur Herstellung von Gewebe-Plasminogen-Aktivator in einem Säugetierwirt, wobei das für den Gewebe-Plasminogen-Aktivator codierende Gen unter der Transkriptionskontrolle eines anderen als dem natürlich vorkommenden Promoter Bereich steht, dadurch gekennzeichnet, daß für die Expression ein Gen mit einer für den Gewebe-Plasminogen-Aktivator codierten Codierungssequenz verwendet wird, bei dem die Codierungssequenz durch mindestens ein Intron von mindestens ungefähr 100 bp unterbrochen ist, und welcher zur Teilung in dem Wirt unter Erzeugung einer mRNA mit dem korrekten Leseraster in der Lage ist.

2. Verfahren nach Anspruch 1 bei dem das Gen ein Chimären-Gen ist, welches cDNA und genomische DNA mit mindestens einem Intron enthält und welches eine vollständige Codierungssequenz mit dem korrekten Leseraster nach der Teilung enthält.

3. Verfahren nach Anspruch 3, bei dem der Gewebe-Plasminogen-Aktivator vom menschlichen Typus ist, und daß ein *Eco*RI—*Eco*RI-genomisches Fragment in dem *Eco*RI—*Eco*RI-Bereich der cDNA ersetzt ist, welche für den menschlichen Gewebe-Plasminogen-Aktivator codiert.

4. Verfahren nach Anspruch 1, bei dem die Intronen natürliche Intronen an den ursprünglichen Stellen sind.

5. Verfahren nach Anspruch 4, wobei ein Promoter-Bereich eines Virus die Transkription des Gens reguliert.

6. DNA-Konstrukt, welches Transkriptions-Start- und Terminations-Regulations-Sequenzen, die in Säugetierwirten wirksam sind, umfaßt und wobei mindestens die Startsequenz von dem Wildtyp der Gewebe-Plasminogen-Aktivator-Startsequenz verschieden ist und wobei ein Gen eine Codierungssequenz aufweist die für den Gewebe-Plasminogen-Aktivator codiert, wobei das Gen durch mindestens ein Intron unterbrochen ist, welches zur Teilung in dem Säugetierwirt zu einer mRNA fähig ist, die das korrekte Leseraster aufweist.

8

7. DNA-Konstrukt nach Anspruch 6, wobei jedes der Intronen ein natürlich vorkommendes Intron an seiner natürlichen position ist.

8. DNA-Konstrukt nach Anspruch 7, bei dem der Gewebe-Plasminogen-Aktivator vom menschlichen Typ ist und bei dem ein *Eco*RI-*Eco*RI-genomisches Fragment in dem *Eco*RI-*Eco*RI-Bereich der cDNA eingefügt ist, die für den Gewebe-Plasminogen-Aktivator codiert.

9. In einem Säugetierwirt stabil erhaltbarer Expressions-Vektor in einem Säugetierwirt, welcher ein DNA-Konstrukt gemäß Anspruch 6 umfaßt.

10. In einem Säugetierwirt stabil erhaltbarer Expressions-Vektor, welcher ein DNA-Konstrukt gemäß Anspruch 7 umfaßt.

11. In einem Säugetierwirt stabil erhaltbarer Expressions-Vektor, welcher ein DNA-Konstrukt gemäß Anspruch 8 umfaßt.

12. Säugetierzelle in Kultur, welche einen Expressions-Vektor gemäß Anspruch 9 umfaßt.

13. Säugetierzelle in Kultur, welche einen Expressions-Vektor gemäß Anspruch 11 umfaßt.

14. Säugetierzelle in Kultur, mit einem in ein Chromosom integriertes DNA-Konstrukt gemäß Anspruch 6.

15. Säugetierzelle in Kultur, mit einem in ein Chromosom integriertes DNA-Konstrukt gemäß Anspruch 8.

## Revendications

1. Dans une méthode pour la production de l'activiteur tissulaire du plasminogène dans un hôte mammifère, dans laquelle le gène codant pour l'activateur tissulaire du plasminogène est sous le contrôle de transcription d'une région promotrice autre que la région naturelle, le perfectionnement comprenant:

l'utilisation pour l'expression, d'un gène ayant une séquence codante, codant pour l'activateur tissulaire du plasminogène, dans lequel la séquence codante est interrompue par au moins un intron d'au moins environ 100bp et est capable d'être coupée dans le susdit hôte pour donner un ABNm ayant le bon cadre de lecture.

2. Méthode selon la revendication 1 dans laquelle le dit gène est un gène chimère comportant de l'ADNc et de l'ADN génomique ayant au moins un intron et donnant une séquence codante complète avec le bon cadre de lecture après épissage.

3. Méthode selon la revendication 2 dans laquelle l'activateur tissulaire du plasminogène est humain et un fragment génomique *Eco*RI-*Eco*RI est substitué dans la région *Eco*RI-*Eco*RI de l'ADNc codant pour l'activateur tissulaire du plasminogène humain.

4. Méthode selon la revendication 1 dans laquelle les introns sont des introns natifs ou sites natifs.

5. Méthode selon la revendication 4 ayant une région promotrice virale pour la régularisation de la transcription dudit gène.

6. Construction d'ADN comprenant des séquences régulatrices de l'initiation et de la terminaison de transcription, fonctionnelles dans un hôte mammifère, la séquence d'initiation au moins étant différente de la séquence d'initiation de type sauvage de l'activateur tissulaire du plasminogène et un gène ayant une séquence codante, codant pour l'activateur tissulaire du plasminogène, dans laquelle le gène est interrompu par au moins un intron capable d'être épissé dans le dit hôte mammifère pour donner un ARNm ayant le bon cadre de lecture.

7. Construction d'ADN selon la revendication 6 dans laquelle chacun des 10 introns est un intron existant naturellement, au niveau de son site naturel.

8. Construction d'ADN selon la revendication 7 dans laquelle le dit activateur tissulaire du plasminogène est humain et un fragment génomique *Eco*RI-*Eco*RI est substitué à la région *Eco*RI-*Eco*RI d-un ADNc codant pour l'activateur tissulaire du plasminogène.

9. Vecteur d'expression capable d'être maintenu de façon stable dans un hôte mammifère comprenant une construction d'ADN selon la revendication 6.

10. Vecteur d'expression capable d'être maintenu de façon stable dans un hôte mammifère comprenant une construction d'ADN selon la revendication 7.

11. Vecteur d'expression capable d'être maintenu de façon stable dans un hôte mammifère comprenant une construction d'ADN selon la revendication 8.

12. Cellule de mammifère en culture contenant un vecteur d'expression selon la revendication 9.

13. Cellule de mammifère en culture contenant un vecteur d'expression selon la revendication 11.

14. Cellule de mammifère en culture dans laquelle la construction d'ADN selon la revendication 6 est intégrée dans un chromosome.

15. Cellule de mammifère en culture dans laquelle une construction d'ADN selon la revendication 8 est intégrée dans un chromosome.

FIG._1.